# EUROPÄISCHE PATENTSCHRIFT

(11) **EP 3 621 535 B1**
(45) Veröffentlichungstag und Bekanntmachung des Hinweises auf die Patenterteilung: **03.03.2021**
(21) Anmeldenummer: 18725445.3
(22) Anmeldetag: 09.05.2018
(51) Int. Cl.: A61B 17/29, A61B 18/14, A61B 17/00, A61B 90/00

(54) **CHIRURGISCHES INSTRUMENT MIT VERBESSERTER SCHLIESSCHARAKTERISTIK**
SURGICAL INSTRUMENT WITH IMPROVED CLOSURE CHARACTERISTICS
INSTRUMENT CHIRURGICAL AVEC DES CARACTERISTIQUES DE FERMETURE AMÉLIORÉES

(30) Priorität: 09.05.2017 DE 102017109891
(43) Veröffentlichungstag der Anmeldung: 18.03.2020
(73) Patentinhaber: Aesculap AG, 78532 Tuttlingen (DE)
(72) Erfinder: WALBERG, Erik, 78532 Tuttlingen (DE); HERNER, Eugen, 78054 Villingen-Schwenningen (DE)
(74) Vertreter: Winter, Brandl, Fürniss, Hübner, Röss, Kaiser, Polte - Partnerschaft mbB
(86) Internationale Anmeldenummer: PCT/EP2018/062010
(87) Internationale Veröffentlichungsnummer: WO 2018/206648

(56) Entgegenhaltungen:
- EP-A1- 2 732 778
- WO-A1-95/15124
- WO-A1-98/51179
- WO-A1-2006/071121
- DE-A1- 3 709 706
- DE-A1-102014 100 603
- DE-C- 293 929
- DE-U1- 29 713 490
- US-A1- 2006 217 697

## Beschreibung

Die Erfindung betrifft ein chirurgisches Instrument, insbesondere elektrochirurgisches Instrument, mit einem Instrumentenschaft, zwei daran distal angeordneten zueinander zwischen einer Arbeitsstellung und einer Ruhestellung relativpositionierbaren /beweglichen Instrumentenbranchen und einem Griffelement, an dem ein manuell betätigbares Bedienelement zum Positionieren der Instrumentenbranchen zwischen der Arbeitsstellung und der Ruhestellung beweglich angeordnet ist. Beispiele für solche Instrumente sind Klemmen, Zangen und RF-Instrumente zur Verödung und/oder Koagulation von Gewebe.

Es sind zum Beispiel chirurgische Instrumente bekannt, die mittels eines zangen- oder scherenartigen Werkzeuges ein Greifen, Halten sowie Klemmen von Körpergewebe ermöglichen, um dieses durch Anlegen einer Hochfrequenzspannung monopolar oder bipolar zu koagulieren oder zu durchtrennen. Klemmbackenartige Instrumentenbranchen müssen dabei in bestimmungsgemäßer Weise Druck auf das zwischen ihnen liegende Gewebe ausüben. Zum Erreichen eines gewünschten Behandlungsergebnisses darf dieser Druck weder zu groß (Zerstören von Gewebe) noch zu klein (unzureichendes Zusammenfügen von Gewebe) sein.

Aus der DE 10 2012 110 660 A1 ist ein chirurgisches HF-Instrument mit zwei Gewebebranchen bekannt, von denen zumindest eine relativ zur anderen bewegbar und über einen Betätigungsmechanismus unter dazwischen Einklemmen von Körpergewebe mit einem vorbestimmten oder vorbestimmbaren Anpressdruck an die andere Gewebebranche anlegbar ist. Das Instrument weist eine Klemmdruck-Regeleinrichtung auf, die in einem Kraft- oder Momentübertragungszug zwischen dem Betätigungsmechanismus und der zumindest einen bewegbaren Gewebebranche zwischengeschaltet ist. Der relevante vorbekannte Stand der Technik ergibt sich aus der WO 2006/071121, DE 10 2014 100603, DE 293 929, WO 95/15124 und WO 98/51179.

Ein weiterer wichtiger Aspekt bei solchen Instrumenten ist die Anwendungs- und Bedienfreundlichkeit. Besonders zu nennen ist in diesem Zusammenhang die durch einen Anwender bei einer Verwendung des Instruments aufzubringende Betätigungskraft. Die Instrumente sollen mit geringen Betätigungskräften präzise zu bedienen sein, um eine Ermüdung des Anwenders, in der Regel eines Operateurs, sowie ggf. auftretende Nebeneffekte wie Zittern der das Instrument bedienenden Hand des Operateurs bei langem Halten oder relativ hohen Haltekräften gering zu halten. Des Weiteren sind geringe und für den Anwender vorausschaubare, also diesen nicht überraschende Betätigungskräfte förderlich für eine exakte, präzise und ruhige Handhabung des Instruments.

Schließlich spielt in der minimalinvasiven Chirurgie bzw. Endoskopie, insbesondere Laparoskopie, die Bauraum- und Gewichtsoptimierung der dort verwendeten chirurgischen Instrumente eine wichtige Rolle. So ist nicht nur bei dem distalen Instrumentenkopf, sondern auch bei dem vom Anwender betätigten proximalen Instrumentengriff auf eine platz- und gewichtssparende Ausgestaltung zu achten.

Die der Erfindung vor diesem Stand der Technik zugrundeliegende Aufgabe ist, ein chirurgisches Instrument zu schaffen, das die vorstehend beschriebenen Eigenschaften verbessert, insbesondere einfach und mit geringen Betätigungs- und Haltekräften zu verwenden ist und/oder eine vorzugsweise einstellbare Überlastsicherung zur Vermeidung von Gewebeschädigungen und/oder zum Schutz der Mechanik bzw. mechanischer Bauteile vor Überbelastung ermöglicht und/oder einfach aufgebaut ist, vorzugsweise mit geringem Gewicht und Bauraum.

Diese Aufgabe wird nach der Erfindung gelöst durch ein chirurgisches Instrument, insbesondere ein elektrochirurgisches Instrument (RF-Instrument), mit den Merkmalen des Anspruchs 1 und insbesondere mit einem Instrumentenschaft, zwei daran distal angeordneten zueinander zwischen einer Arbeitsstellung und einer Ruhestellung relativpositionierbaren Instrumentenbranchen, einem Griffelement/Griffabschnitt, an dem ein insbesondere manuell betätigbares Bedienelement zum wahlweisen Positionieren der Instrumentenbranchen in der Arbeitsstellung und der Ruhestellung beweglich angeordnet ist, und einem Koppelmechanismus mit einer eine Bewegung des Bedienelements (infolge einer nutzerseitigen Betätigung) nicht linear in eine Relativbewegung zumindest einer der beiden Instrumentenbranchen wandelnden Übersetzungseinheit, wobei der Koppelmechanismus eine Öffnungs- und/oder Schließ-Betätigungskraft-Unterstützungseinheit, vorzugsweise zumindest ein Vorspannelement aufweist oder hat, das ihn in die Ruhestellung (Offenstellung) und/oder in die Arbeitsstellung (Schließstellung) der Instrumentenbranchen zumindest temporär vorspannt.

Nach der Erfindung sind die Instrumentenbranchen zwischen der Arbeitsstellung und einer Ruhestellung relativpositionierbar. In der Arbeitsstellung können die Instrumentenbranchen geschlossen sein, dann sind sie in der Ruhestellung offen, zum Beispiel im Falle einer Gewebeklemme oder eines Koagulationsinstruments. Alternativ können sie in der Arbeitsstellung offen und in der Ruhestellung geschlossen sein, zum Beispiel im Falle eines Spreizinstruments. Während die Instrumentenbranchen distal am Instrumentenschaft angeordnet sind, ist das Griffelement vorzugsweise proximal daran angeordnet oder ausgebildet. Das Griffelement kann nach der Erfindung als Handgriff, insbesondere als einen Handgriff ausbildendes Gehäuse, ausgebildet sein. Alternativ kann das Griffelement in Form eines Roboterarms bzw. in Form eines Interface zum mechanischen/hydraulischen Anschließen eines Roboterarms ausgebildet sein. In dem Griffelement als Gehäuse können Teile des Bedienelements, das Übertragungselement, ein im weiteren Verlauf der Beschreibung erwähntes Federelement sowie der Kopplungsmechanismus oder Teile davon aufgenommen, eingehaust und gelagert sein.

Das Bedienelement kann insbesondere positionierbar und/oder schwenkbar am Griffelement angeordnet sein, zum Beispiel in Form eines Betätigungshebels oder Schiebers. Vorzugsweise ist es einerseits mit dem Griffelement und andererseits mit dem Koppelmechanismus wirktechnisch verbunden. Vorzugsweise wird durch eine anwenderseitige Betätigung des Bedienelements der Koppelmechanismus oder ein Teil des Koppelmechanismus gegenüber dem Griffelement relativpositioniert, insbesondere in Axialrichtung des Instrumentenschafts translatorisch positioniert.

Die Instrumentenbranchen am distalen Ende des Schafts sind eingerichtet und bestimmt, um Gewebe zu klemmen und/oder zu schneiden und/oder mit Energie zu beaufschlagen, insbesondere mit hochfrequentem Strom zum Koagulieren, Veröden oder Schneiden des Gewebes.

Der Koppelmechanismus ist nach der Erfindung derart ausgebildet, dass das Instrument (bzw. dessen vom Koppelmechanismus gebildete Getriebe bzw. dessen Kraftübertragungszug) ein nicht-lineares Übertragungsverhalten aufweist. Das bedeutet, dass das Verhältnis von Betätigungskraft des Bedienelements zur hervorgerufenen Schließkraft der Instrumentenbranchen und/oder von Betätigungsweg des Bedienelements zur hervorgerufenen Relativpositionierung (z. B. Öffnen/Schließen) der Instrumentenbranchen nicht linear ist. Vorzugsweise ist das Übertragungsverhalten derart, dass ein anfänglicher Verstellweg (1. Drittel - 1. Viertel) des Bedienelements (anfänglich im Sinne von ausgehend von der Ruheposition) zu einer relativ großen Relativpositionierung (2/3 - 3/4) der Instrumentenbranchen führt, sich das Verhältnis kontinuierlich im Verstellwegsverlauf ändert und ein schlussendlicher Verstellweg (letztes Drittel - Viertel) des Bedienelements (schlussendlich im Sinne von kurz vor der Arbeitsposition) zu einer relativ kleinen Relativpositionierung (1/3 - 1/4) der Instrumentenbranchen führt. Außerdem kann das Übertragungsverhalten derart beschaffen sein, dass eine anfängliche auf das Bedienelement ausgeübte Betätigungskraft bzw. ein anfängliches auf das Bedienelement ausgeübtes Betätigungsmoment (anfänglich im Sinne von ausgehend von der Ruheposition) zu einer relativ geringen resultierenden Kraft und/oder zu einem relativ geringen Moment der Instrumentenbranchen führt, sich das Verhältnis "Betätigungs-/Schließkraft" fortlaufend ändert und eine schlussendliche Betätigungskraft bzw. ein schlussendliches Betätigungsmoment (schlussendlich im Sinne von kurz vor der Arbeitsposition) zu einer relativ großen resultierenden Kraft und/oder zu einem relativ großen resultierenden Moment der Instrumentenbranchen führt. Das vorstehend beschriebene Übertragungsverhalten wird durch die Implementierung der Übersetzungseinheit (Kniehebel-Prinzip) in den Koppelmechanismus erzielt. Die Übersetzungseinheit ist zum einen schwenkbar, insbesondere unmittelbar, an dem Bedienelement angelenkt und wirkt zum anderen mit zumindest einer der Instrumentenbranchen zusammen, zumindest mittelbar über weitere zwischenliegende Elemente des Koppelmechanismus.

Erfindungsgemäß ist der Koppelmechanismus mittels zumindest einem Vorspannelement der ersten Art (temporär) vorgespannt. Durch seine Vorspannung insbesondere in die Ruheposition kann eine Rückstellung der Instrumentenbranchen aus der Arbeitsstellung mit geringerer Betätigungskraft bewirkt werden. Durch eine zusätzliche Vorspannung insbesondere in die Arbeitsposition kann das Instrument mit einer Art Überlastsicherung/Weg-Begrenzung/Kraft-Begrenzung versehen werden. Wichtig ist, dass das Vorspannelement der ersten Art oder die Vorspannelemente Bestandteil des Koppelmechanismus ist bzw. sind und damit in dessen Kraftfluss angeordnet ist bzw. sind. Dadurch wird gegenüber bekannten Instrumenten der Vorteil erzielt, dass die Vorspannung nicht unmittelbar, sondern nur mittelbar, auf das Bedienelement wirkt, also auch dem Übertragungsverhalten des Koppelmechanismus unterliegt. Daher wirken Kräfte/Momente zwischen dem Vorspannelement und dem Bedienelement mit gleicher/ähnlicher Übersetzung wie Kräfte/Momente zwischen den Instrumentenbranchen und dem Bedienelement. Die haptische Wahrnehmung bei der Handhabung des Instruments wird somit nicht durch dessen Vorspannung verfälscht. Der Koppelmechanismus kann insbesondere zumindest zwei Vorspannelemente aufweisen, von denen ein erstes (der ersten Art) in die Arbeitsstellung (temporär) vorspannt und ein zweites (der zweiten Art) in die Ruhestellung (temporär) vorspannt. Zum Übertragen von Betätigungskräften vom Bedienelement auf die Instrumentenbranchen kann die Koppeleinheit insbesondere eine durch den Schaftabschnitt hindurchragende Zug-Druck-Einheit (Zug-Druck-Stange) aufweisen. Diese kann zum Beispiel gebildet sein aus einem Hohlprofil (Rohr) als Druckelement und einem darin angeordneten und zu diesem axialverschiebbaren Zugelement (Seil/Stange). Derartige Zug-Druck-Einheiten sind allgemein bekannt, so dass vorliegend auf eine weitere diesbezügliche Beschreibung verzichtet wird.

Das Instrument kann somit eine Rückstellunterstützung für den Anwender beinhalten, was jedoch keinen Einfluss auf die letztlich zum bestimmungsgemäßen Betätigen des Instruments erforderliche Kraft hat. Insbesondere kann das Übertragungsverhalten des Instruments derart ausgebildet sein, dass die Wirkung dieser Rückstellunterstützung in einem Betätigungsbereich relativ nahe der Arbeitsposition mehr oder weniger geschwächt oder sogar aufgehoben ist, während sie in einem Bereich nahe der Ruheposition nur unwesentlich vermindert oder unvermindert ist. Dies erleichtert ein Halten (Bewahren) des Instruments in der Arbeitsposition. Man kann auch sagen, dass das Verhältnis der auf das Bedienelement wirkenden Rückstellkraft zur durch den Anwender auf das Bedienelement zum Überwinden der Rückstellkraft aufzubringenden Betätigungskraft in einem Bereich nahe der Arbeitsposition oder in der Arbeitsposition kleiner ist als dieses Verhältnis in einem Bereich nahe der Ruheposition oder in der Ruheposition.

Des Weiteren kann das Instrument eine Überlastsicherung aufweisen. Diese kann durch das Vorspannelement (der ersten Art) oder eines der Vorspannelemente bewirkt werden, das bzw. die entsprechende elastische Eigenschaften aufweist bzw. aufweisen und in den Koppelmechanismus integriert ist bzw. sind. Es ist aber von besonderem Vorteil, wenn die Überlastsicherung durch das Übertragungselement bewirkt wird, das zu diesem Zweck mit den entsprechend gewünschten elastischen Eigenschaften versehen ist und eine Art Feder bildet, die in den Koppelmechanismus integriert ist. Das Vorspannelement (der ersten Art) bzw. das Übertragungselement steht nach der Erfindung nicht zwingend unter einer (permanenten) Vorspannung. Es weist jedoch elastische Federeigenschaften auf. Bei Überschreiten einer gewissen Belastung, insbesondere eines durch eine Vorspannung voreingestellten Grenzwerts, durch Kraftbeaufschlagung mittels des Bedienelements federt das Vorspannelement bzw. das Übertragungselement aufgrund seiner elastischen Eigenschaften ein. Daher wird eine weitere Betätigung des Bedienelements nur noch mittelbar über die Verformung des Vorspannelements bzw. des Übertragungselements auf die Instrumentenbranchen übertragen und eine Überbelastung von zwischen den Branchen vorliegendem Gewebe und/oder eine Überbelastung von anderen mechanischen Bauteilen der Betätigungsmechanik kann vermieden werden.

Vorteilhafte Ausführungsformen der Erfindung sind auch in den Unteransprüchen beansprucht und werden nachfolgend näher erläutert.

Eine Ausführungsform der Erfindung ist dadurch gekennzeichnet, dass der Koppelmechanismus mit einem Vorspannelement (der zweiten Art), insbesondere einem Federelement, insbesondere in die Ruhestellung, vorzugweise in die Offenstellung der Instrumentenbranchen, vorgespannt ist. Das Federelement ist auf der in Richtung des Kraftflusses dem Bedienelement abgewandten Seite der Übersetzungseinheit angeordnet. Es bildet einen besonders einfachen und robusten Mechanismus aus, über den das Instrument mit einer Rückstellunterstützung versehen werden kann. Das Federelement kann zum Beispiel als Blattfeder ausgebildet sein. Es ist vorzugsweise einerseits ortsfest zu den Instrumentenbranchen gehalten, insbesondere an dem Griffelement gelagert (ein Blattfederende ist am Griffgehäuse gehalten/fixiert), und andererseits mit einem Koppelelement gekoppelt, welches über die Zug-Druck-Einheit mit zumindest einer der beiden Instrumentenbranchen verbunden ist. Zur Kopplung mit dem Koppelelement kann das Federelement nach einer Ausführungsform der Erfindung eine, insbesondere gabelförmige, Aufnahme aufweisen. Diese steht mit einer zu diesem Zweck ausgebildeten Koppelkontur (Hinterschnitt/Nut) des Koppelelements in Eingriff. Die Koppelkontur kann insbesondere als umlaufende Nut ausgebildet sein. Die gabelförmige Aufnahme kann so das Zug-Element bzw. das Koppelelement in der Ausnehmung zwischen den Gabelarmen aufnehmen und mit den Gabelarmen beidseitig in die Nut eingreifen, so dass eine Krafteinleitung in das Koppelelement in rein axialer Richtung sichergestellt ist.

Nach einer Ausführungsform kann das Federelement vorgespannt sein, insbesondere bei in der Arbeitsstellung befindlichen Instrumentenbranchen in die Ruhestellung vorspannt sein oder bei in der Ruhestellung befindlichen Instrumentenbranchen in die Arbeitsstellung vorgespannt sein. Auf diese Weise kann eine gewünschte Rückstellkraft bewirkt werden, die nicht nur in der Arbeitsstellung der Instrumentenbranchen, sondern auch nahe der Ruheposition oder sogar in der Ruheposition wirkt und stets ein vollständiges Rückstellen des Instruments in die Ruhestellung sicherstellt. Die Vorspannung des Federelements kann anwenderseitig einstellbar sein. Beispielsweise kann für die federelastische Einstellung Vorspannung des Federelements ein zusätzliches Einlegeelement, vorzugsweise ein U-geformtes Blech, oder können entsprechende Versteifungen vorgesehen sein.

Eine weitere Ausführungsform ist dadurch gekennzeichnet, dass die Übersetzungseinheit im Kraftfluss zwischen dem Bedienelement und dem Federelement angeordnet ist. Die Übersetzungseinheit ist nach der Erfindung vorzugsweise in Form eines Kniehebelelements ausgebildet. Andere Ausführungsformen der Übersetzungseinheit sind möglich und liegen im Bereich der Erfindung, wie zum Beispiel eine Nockeneinheit (Cam) oder ein Vier-Gelenk-Gestänge.

Nach einer Ausführungsform weist der Koppelmechanismus ein Kniehebelelement als Übersetzungseinheit auf. Dieses ist vorzugsweise zum einen schwenkbar unmittelbar an dem Bedienelement angelenkt und wirkt zum anderen mittelbar mit zumindest einer der Instrumentenbranchen zusammen, zum Beispiel über ein axialpositionierbares Zug-Element (Band) des Kopplungsmechanismus, das mit zumindest einer der Instrumentenbranchen zu deren Relativpositionierung gekoppelt ist. Vorzugsweise weist das Kniehebelelement einerseits eine Lagerkontur zur schwenkbaren Anlenkung an dem Bedienelement und andererseits einen Koppelabschnitt mit zwei beiderseits einer zentralen Ausnehmung ausgebildeten Koppelarmen auf. Auf diese Weise ist eine beidseitige Kopplung von Kniehebelelement und Koppelmechanismus möglich, so dass Betätigungskräfte in rein axialer Richtung eingeleitet werden.

Es ist besonders vorteilhaft, wenn das Kniehebelelement, das insbesondere als Blechformteil und/oder Blechstanzteil ausgebildet ist, eine im Wesentlichen U-förmige Aufnahme zur Kopplung mit dem Federelement aufweist. Das Federelement kann ebenfalls eine im Wesentlichen U-förmige Koppelaussparung zur Kopplung mit dem Kniehebelelement aufweisen. Das Federelement und das Kniehebelelement werden bzw. sind derart zueinander angeordnet, dass ihre jeweiligen Koppelaussparungen ineinander greifen und eine dynamische Kopplung mit den zum bestimmungsgemäßen Gebrauch erforderlichen Freiheitsgraden ermöglichen. Vorzugsweise ist die Koppelaussparung des Kniehebelelements auf der der zentralen Ausnehmung zugewandten Innenseite der Koppelarme ausgebildet.

Eine besondere Ausführungsform der Erfindung ist dadurch gekennzeichnet, dass das Übertragungselement einen im Wesentlichen U-förmigen Federabschnitt mit zwei einander gegenüberliegenden Federarmen aufweist. Über den Federabschnitt kann das Übertragungselement besonders einfach mit entsprechenden elastischen Eigenschaften versehen werden, die zum Bewirken der vorstehend bereits beschriebenen Funktion einer Überlastsicherung erforderlich sind. Ein solches Federelement ist in vorteilhafter Weise klein, sprich erfordert einen nur geringen Bauraum, ist robust und stellt Federeigenschaften in einer Richtung quer zu den beiden Federarmen sicher, während in die übrigen Richtungen eine ausreichend hohe Steifigkeit gewährleistet ist. Es ist von besonderem Vorteil, wenn die Vorspannung des Federabschnitts durch eine die Federarme gegeneinander verspannende Stellschraube einstellbar ist. Auf diese Weise kann die derart bewirkte Überlastsicherung schnell und einfach auf einen bestimmten gewünschten Wert eingestellt werden.

Die Erfindung wird nachfolgend beispielhaft mit Hilfe von Zeichnungen näher erläutert. Es zeigt dabei:
Figur 1 eine Schnittansicht eines Abschnitts eines chirurgischen Instruments nach der Erfindung in einer ersten Funktionsstellung,
Figur 2 die Schnittansicht der Figur 1, wobei zum besseren Verständnis Teile des Instruments nicht dargestellt sind,
Figur 3 eine Schnittansicht eines Abschnitts eines chirurgischen Instruments nach der Erfindung in einer zweiten Funktionsstellung,
Figur 4 die Schnittansicht der Figur 3, wobei zum besseren Verständnis Teile des Instruments nicht dargestellt sind,
Figur 5 eine Schnittansicht eines Abschnitts eines chirurgischen Instruments nach der Erfindung in einer dritten Funktionsstellung,
Figur 6 die Schnittansicht der Figur 5, wobei zum besseren Verständnis Teile des Instruments nicht dargestellt sind,
Figur 7 in einem Ausschnitt eine perspektivische Darstellung eines Teils des Koppelmechanismus mit Übertragungseinheit sowie des Federelements,
Figur 8 den Ausschnitt der Figur 7 aus einer anderen Perspektive,

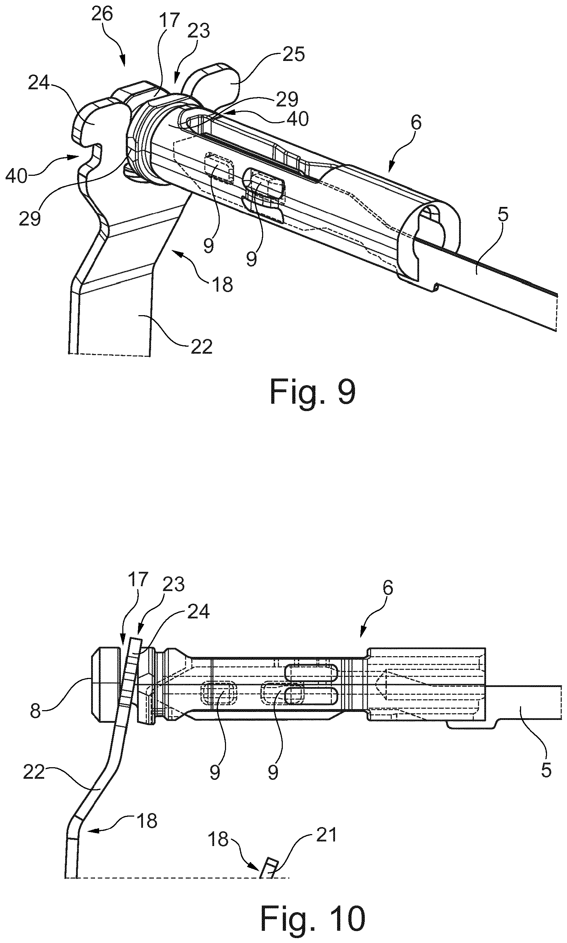

Figur 9 in einem Ausschnitt eine perspektivische Darstellung eines Teils des Koppelmechanismus sowie des Federelements,
Figur 10 den Ausschnitt der Figur 9 in einer seitlichen Ansicht,

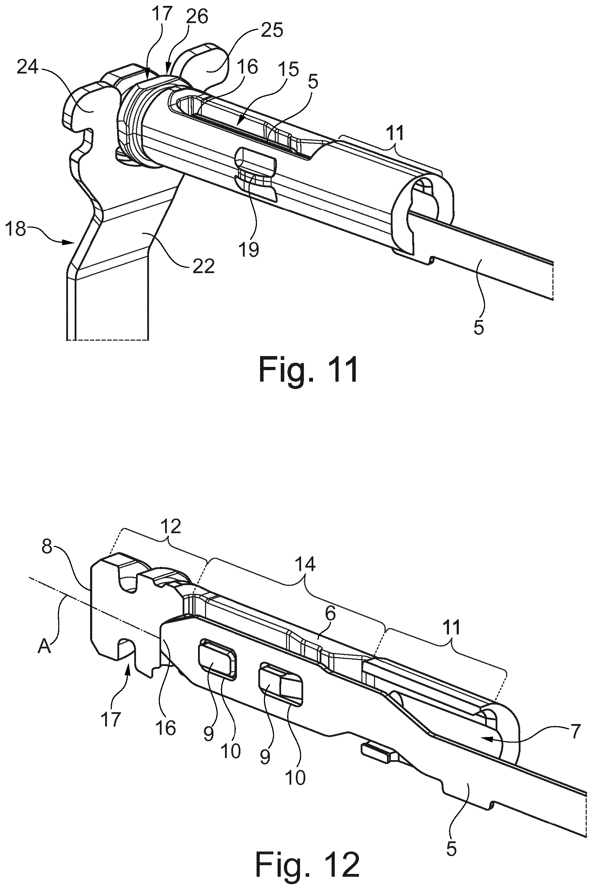

Figur 11 in einem Ausschnitt eine perspektivische Darstellung eines Teils des Koppelmechanismus,
Figur 12 den Ausschnitt der Figur 11 in einem Schnitt,

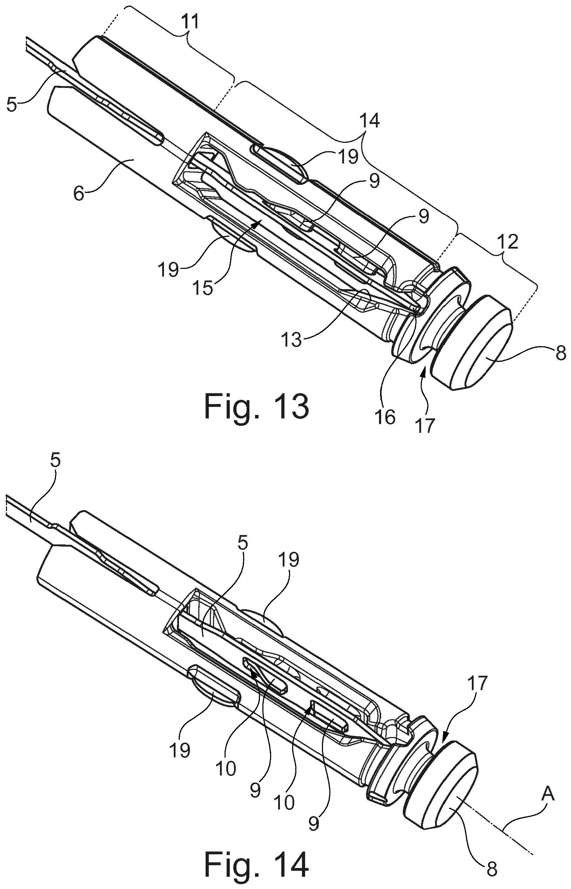

Figur 13 einen Teil des Koppelmechanismus in einer perspektivischen Ansicht,
Figur 14 den Ausschnitt der Figur 13 aus einer anderen Perspektive und
Figur 15 den Ausschnitt der Figuren 13 und 14 in einer seitlichen Ansicht.

Die Zeichnungen sind lediglich schematischer Natur und dienen nur dem Verständnis der Erfindung.

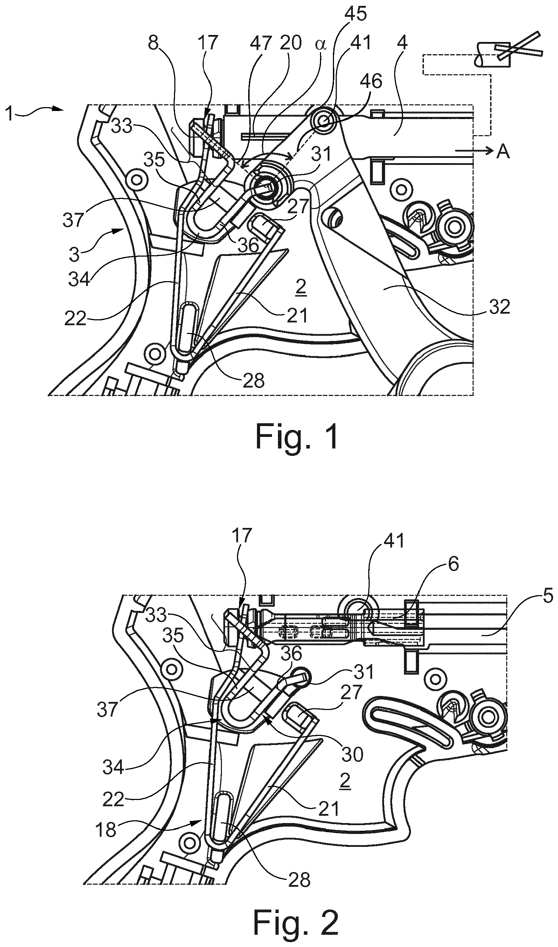

Das beispielhaft in den Figuren gezeigte chirurgische Instrument 1 weist ein vorzugsweise aus zwei Schalen/Hälften aufgebautes (Griff-)Gehäuse 2 auf, von dem in den Schnittdarstellungen der Figuren 1 bis 6 nur die Innenseite der einen/linken Gehäusehälfte (bei Betrachtung von einer das Instrument 1 bestimmungsgemäß haltenden Person aus) dargestellt ist. Die in den Figuren nicht gezeigte andere/rechte Gehäusehälfte ist zur linken Gehäusehälfte spiegelsymmetrisch. Das Gehäuse 2 ist als/ mit einem Griffelement/Griffabschnitt 3 oder Handgriff 3 ausgebildet. Die proximale Seite des Instruments 1 befindet sich in den Figuren 1 bis 6 links, die distale Seite rechts. Das Griffelement 3 befindet sich also in einem proximalen unteren Bereich des Gehäuses 2.

Im (proximalen) Gehäuse 2 ist ein Schaft/Instrumentenschaft 4 zum Gehäuse 2 lagefixiert, insbesondere in dessen Axialrichtung A fixiert, angeordnet. Der Schaft 4 ist im Wesentlichen in Form eines Hohlschafts/Rohrs ausgebildet und erstreckt sich aus dem Gehäuse 2 hinausragend in distaler Richtung (dies ist in den Figuren nicht näher zu erkennen). An seinem in den Figuren nicht näher gezeigten distalen Ende trägt der Schaft 4 zwei Instrumentenbranchen, die axialfest aber schwenkbar am Schaft 4 angelenkt sind. Wie insbesondere aus den Figuren 2, 4 und 6 hervorgeht, ist im Inneren des Schafts 4 ein Zug-Element 5 z. B. in Form eines Zugbands oder Bowdenzugs 5 angeordnet. Das Zug-Element 5 ist relativ zum Schaft 4 axialpositionierbar und an seinem in den Figuren nicht näher gezeigten distalen Ende mit zumindest einer der Instrumentenbranchen verbunden. Mit seinem proximalen Endabschnitt greift das Zugband 5 formschlüssig in ein Koppelelement in Form einer Koppelhülse 6 ein, die ebenfalls im Inneren des Schafts 4 in dessen Längsrichtung axialpositionierbar angeordnet ist, wie ein Vergleich der unterschiedliche Betriebspositionen zeigenden Figuren 1, 3 und 5 veranschaulicht.

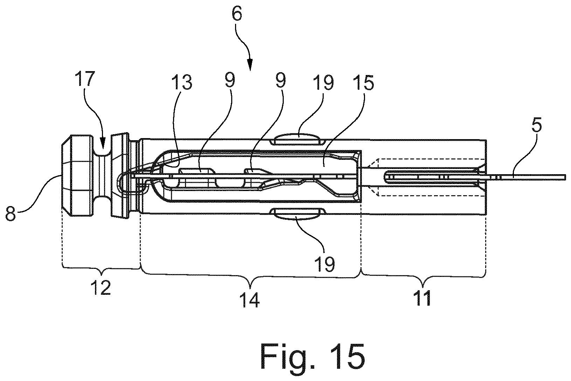

Die Koppelhülse 6 ist durchgängig hohl ausgebildet und weist ein distales, offenes axiales Ende 7 sowie ein proximales, geschlossenes axiales Ende 8 auf. Das Zugband 5 greift durch das offene Ende 7 der Koppelhülse 6 und ist über in deren Inneren ausgebildeten Koppelnasen 9 und über im Zugband 5 ausgebildeten Koppelöffnungen 10 mit der Koppelhülse 6 in axialfester Weise verrastet. Um ein einfaches und sicheres Koppeln von Zugband 5 und Koppelhülse 6 zu erzielen, weist die Koppelhülse 6 (siehe insbesondere in Figur 15) einen distalen inneren Führungsabschnitt 11 auf, dessen Innenquerschnittsform im Wesentlichen der Außenquerschnittsform des Zugbands 5 entspricht, in dem das Zugband 5 seitlich abgestützt und geführt ist. Ein proximaler Endabschnitt 12 der Koppelhülse 6 ist mit einer inneren Führungsschräge 13 versehen, an dem das proximale pfeilspitzförmig gebildete Ende des Zugbands 5 bei bestimmungsgemäßer Anordnung in der Koppelhülse 6 anliegt (siehe Figur 15). In einem (aufgeweiteten) axialen Mittelabschnitt 14 zwischen den Abschnitten 11 und 12 weist die Koppelhülse 6 einen Hohlraum 15 auf, dessen Breite deutlich breiter als die des Zugbands 5 ist und in dem das Zugband 5 seitlich nicht geführt ist. Distal sind die Koppelnasen 9 abgeschrägt ausgebildet, so dass bei einem Einführen des Zugbands 5 von distal nach proximal (in Figur 15 von rechts nach links) das proximale Ende 16 des Zugbands 5 aus der Achsrichtung A abgelenkt wird und an den Koppelnasen 9 vorbei in die Koppelhülse 6 eingeführt werden kann, bis es an die Führungsschräge 13 stößt und dadurch wieder zurück in die Achsrichtung A gedrängt wird, wodurch die Koppelnasen 9 in die Koppelöffnungen 10 eingreifen und verrasten. Auf diese Weise sind das Zugband 5 und die Koppelhülse 6 axialfest miteinander gekoppelt.

Im proximalen Endabschnitt 12 ist eine umlaufende Außen-Nut 17 in die Koppelhülse 6 eingebracht. Diese dient, wie später noch ausführlich beschrieben wird, einer Kopplung der Koppelhülse 6 mit einem Federelement 18 (Vorspannelement der zweiten Art). In ihrem Mittelabschnitt ist die Koppelhülse 6 mit jeweils einer äußeren Führung 19 auf einander gegenüberliegenden Seiten versehen, die in im Schaft 4 ausgebildete Führungsschlitze 20 nach dem Nut-Feder-Prinzip eingreifen, wodurch die Koppelhülse 6 drehfest im Schaft 4 gehalten aber darin axialbewegbar gelagert ist.

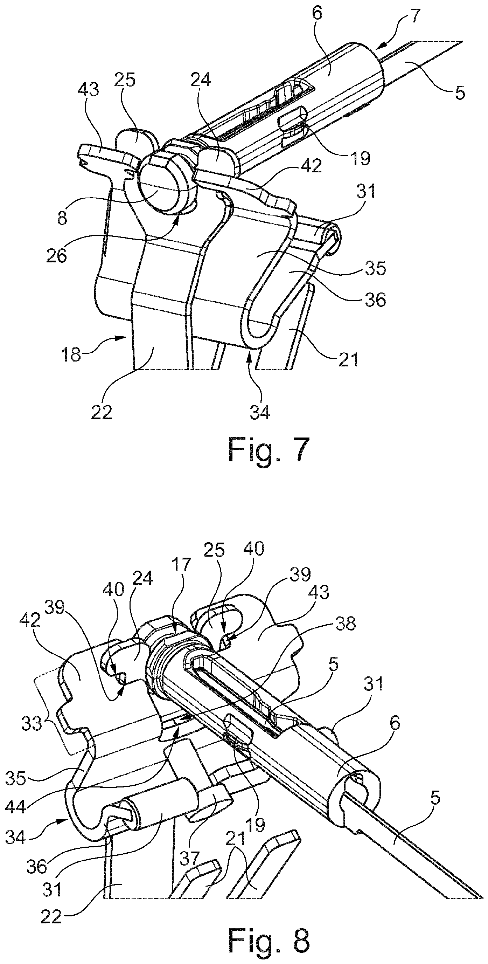

Das Federelement 18 in Form einer Blattfeder ist ein im Wesentlichen U- oder V-förmig geformtes/gebogenes Stanzteil aus Blech. Es weist einen ersten Federarm 21 und einen gegenüber diesem abgewinkelten zweiten Federarm 22 auf. Das dem ersten Federarm 21 gegenüberliegende freie Ende des zweiten Federarms 22 ist als gabelförmige Aufnahme 23 mit zwei Gabelarmen 24 und 25 ausgebildet. Zwischen den beiden Gabelarmen 24 und 25 liegt eine zentrale Ausnehmung 26, die (siehe zum Beispiel in Figur 7) von der Koppelhülse 6 derart durchgriffen ist, dass die Gabelarme 24 und 25 in die Umfangsnut 17 eingreifen. Wie aus den Figuren 1 bis 6 hervorgeht, ist das Federelement 18 (im Abwinkelbereich ihrer U-/V-Form) im Gehäuse 2 gelagert, nämlich über eine lose Anlage des freien Endes des ersten Federarms 21 an einem einen Festlagerpunkt ausbildenden Lagerbock 27 im Gehäuse 2 sowie über einen ein Schwenklager ausbildenden Lagerbock 28 im Knick, bzw. Abwinkel-/Verbindungsbereich beider Federarme 21 und 22. An den jeweiligen Innenseiten der Gabelarme 24 und 25, also an deren der zentralen Ausnehmung 26 zugewandten Seite, ist jeweils ein Zapfen/Vorsprung 29 ausgebildet, der zum eindickenden Koppeln des Federelements 18 mit der Koppelhülse 6 in der umlaufende Nut 17 eingreift. Da die Zapfen 29 in tangentialer Richtung zur Nut (also in Richtung des jeweiligen Gabelarms 24 bzw. 25) eine nur relativ geringe Breite aufweisen, ist das Federelement 18 um ein gewisses Maß gegenüber dem Koppelelement 6 verschwenkbar, was auch aus einem Vergleich der Figuren 1, 3 und 5 hervorgeht.

Der Koppelmechanismus weist des Weiteren ein Übertragungselement 30 nach Art eines Kniehebelelements 30 auf. Das Kniehebelelement 30 ist ein im Wesentlichen U-förmig gestanztes/gebogenes Blechformteil, das an seinem einen Endbereich (in Figur 1 rechts) mit einer Lagerkontur 31, beispielsweise in Form einer Öse oder eines Bolzens 31, versehen ist. Über die Lagerkontur 31 ist das Kniehebelelement 30 relativverschwenkbar an einem später ausführlicher erläuterten Bedienelement 32 angeordnet. Das Kniehebelelement 30 ist an seinem der Lagerkontur 31 gegenüberliegenden Endbereich zu einem Hebelarm 33 ausgebildet. Zwischen dem Hebelarm 33 und der Lagerkontur 31 ist das Kniehebelelement 30 mit einem Federabschnitt 34 (Vorspannelement der ersten Art) mit einem ersten Federarm 35 und einem zweiten Federarm 36 ausgebildet. Der Federabschnitt 34 ist im Wesentlichen U-förmig ausgebildet, so dass sich die beiden Federarme 35 und 36 gegenüberliegen. Der Federabschnitt 34 versieht das Kniehebelelement 30 mit einer gewissen Elastizität, so dass eine Art Überlastsicherung bereitgestellt wird. Denn eine zu hohe Belastung an den Instrumentenbranchen wirkt über den Koppelmechanismus auf den Federabschnitt 34 und führt zu dessen Einfedern, so dass zu hohe Belastungen abgefedert werden. Die Vorspannung des Federabschnitts 34 ist durch eine die Federarme 35 und 36 durchgreifende und gegeneinander verspannende Stellschraube 37 einstellbar. In anderen Worten ausgedrückt ist das federelastische Kniehebelelement 30 aus einem Blechstreifen gefertigt (Blattfederelement), der im Querschnitt in seinem mittleren Federabschnitt 34 U-/V-förmig gebogen ist und so die beiden Federarme 35 und 36 ausbildet. An dem freien Ende des einen (distalen) Federarms 36 ist die Öse oder ein Schwenk-/Scharnierbolzen/-zapfen 31 angeordnet/ausgebildet. An dem freien Ende des anderen (proximalen) Federarms 35 ist eine ca. 90°-Abwinklung ausgeformt, wodurch sich der andere (proximale) Federarm 35 zu dem zungen-/laschenförmigen Hebelarm 33 verlängert, der sich in Richtung proximal erstreckt.

Die beiden Federarme 35 und 36 sind in ihren Mittenabschnitten über die bolzenartige Stellschraube 37 gekoppelt. Hierfür ist in dem einen (distalen) Federarm 36 ein Durchsteckloch und in dem anderen (proximalen) Federarm 35 eine Gewindebohrung ausgeformt. Durch Eindrehen der Stellschraube 37 können so die beiden Federarme 35 und 36 mehr oder weniger gegeneinander (über dem Schraubenkopf) gedrückt und damit die Vorspannung der bügelförmigen Blattfeder eingestellt werden. Wie besonders gut aus den Figuren 7 und 8 hervorgeht, ist im Hebelarm 33 eine im Wesentlichen U-förmige Aufnahme 38 zur umgreifenden, formschlüssigen Aufnahme des Federelements 18 ausgebildet. Die Aufnahme 38 besteht im Wesentlichen aus zwei Koppelarmen 42 und 43, die beiderseits einer zentralen Ausnehmung 44 angeordnet sind. In den beiden der Aufnahme 38 zugewandten Seiten der Koppelarme 42 und 43 des Hebelarms 33 ist jeweils eine Koppelaussparung 39 zur (formschlüssigen) Kopplung mit dem Federelement 18 ausgebildet. Jeweils eine entsprechende Koppelaussparung 40 ist auf den beiden Außenseiten der Gabelarmen 24 und 25 des Federelements 18 ausgebildet. Zur Kopplung von Kniehebelelement 30 und Federelement 18 greifen deren Koppelaussparungen 39 und 40 in der besonders in den Figuren 7 und 8 gezeigten Weise ineinander, so dass das Kniehebelelement 30 in einer relativverschwenkbaren Weise mit dem Federelement 18 gekoppelt ist und trotzdem eine Kraft von der Blattfeder 34 auf die Feder 18 übertragen werden kann.

Ein Bewegen der Instrumentenbranchen erfolgt durch eine anwenderseitige Betätigung eines Bedienelements 32. Dieses ist nach Art eines Abzugshebels ausgebildet und - wie eine vergleichende Betrachtung der Figuren 1 bis 6 ergibt - schwenkbar mittels einer Lagerstruktur 41 (Schwenklager/Scharnier 41) des Gehäuses 2 im Griffelement 3 gelagert. Das Schwenklager 41 ist im Gehäuse 2 fest, also nicht positionierbar, ausgebildet. Das Bedienelement 32 umfasst des Weiteren eine Aufnahme 45 für die Lagerkontur 31 des Kniehebelelements 30 sowie eine aus dem Griffelement 3 herausragende Finger- oder Handöffnung zum betätigenden Ergreifen durch einen Anwender.

Das Bedienelement 32 und das Kniehebelelement 30 sind derart ausgebildet und zueinander konfiguriert, dass sie einen Kniehebelmechanismus ausbilden, mit einem ersten Kniehebelarm 46, der durch das Bedienelement 32 gebildet ist und sich von dessen Schwenklager 41 bis zur Aufnahme 45 erstreckt, und einem zweiten Kniehebelarm 47, der durch das Kniehebelelement 30 gebildet ist und sich von dessen Koppelaussparungen 39 bis zu dessen Lagerkontur 31 erstreckt. Schließlich bildet das Bedienelement 32 einen dritten Hebelarm, der im Wesentlichen rechtwinklig zu dem ersten Kniehebelarm 46 ausgerichtet ist und an seinem freien Ende die Finger- oder Handöffnung definiert/hat.

Die Figuren 1 und 2 zeigen eine Schnittansicht eines Abschnitts des chirurgischen Instruments 1 in der Ruhestellung als erste Funktionsstellung, in der die Instrumentenbranchen offen sind. Für ein besseres Verständnis sind in Figur 2 das Bedienelement 32 und der Schaft 4 nicht dargestellt. In dieser Stellung ist die Koppelhülse 6 mit dem Zug-Band 5 durch die Wirkung des Federelements 18 in die distale Richtung (in den Figuren nach rechts) positioniert. Die Instrumentenbranchen befinden sich in der Ruhestellung und sind vorzugsweise geöffnet. Die beiden Hebelarme 46 und 47 der Kniehebelmechanik sind deutlich zueinander gebeugt (Kniehebelwinkel a beträgt in etwa 90°).

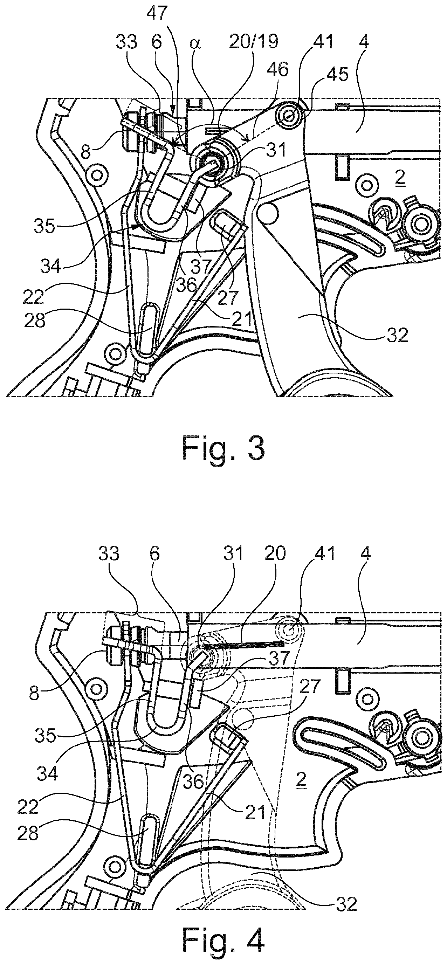

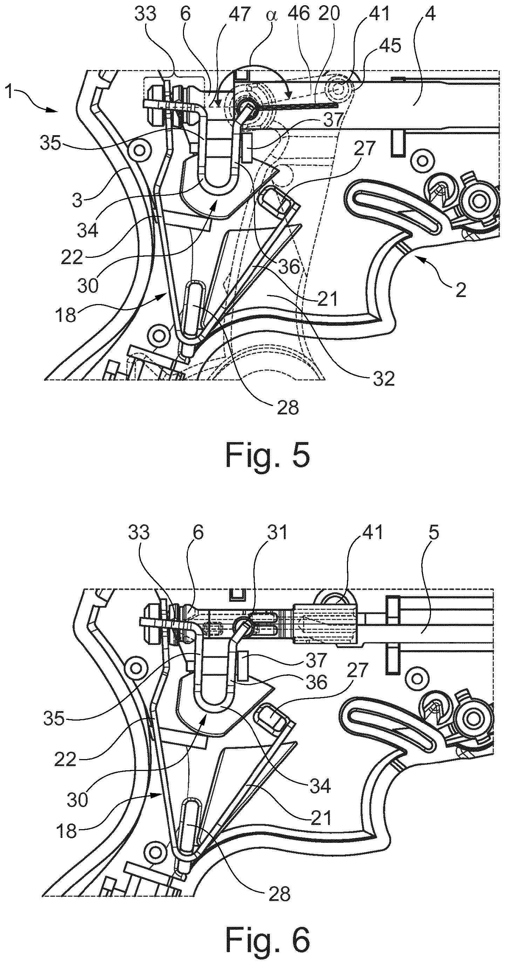

Die Figuren 3 und 4 zeigen Schnittansichten während eines Überführens des Instruments aus der in den Figuren 1 und 2 gezeigten ersten Funktionsstellung (Ruhestellung) in die in den Figuren 5 und 6 gezeigte zweite Funktionsstellung (Arbeitsstellung). Gegenüber der Funktionsstellung der Figur 1 ist das Bedienelement 32 bereits um ein gewisses Maß um sein Schwenklager 41 in Richtung hin zum Griff 3 verschwenkt. Die Hebelarme 46 und 47 der Kniehebelmechanik sind gegenüber Figur 1 mehr gestreckt (geradlinig ausgerichtet), der Kniehebelwinkel a ist größer als in der Stellung der Figuren 1 und 2. Durch die Streckung der Kniehebelmechanik ist es gegenüber der Stellung der Figuren 1 und 2 zu einer Relativpositionierung (Axialverschiebung) der Koppelhülse 6 mit dem Zugband 5 im axialfest zum Griffelement 3 ausgebildeten Schaft 4 in Richtung proximal gekommen. Die Instrumentenbranchen haben dabei bevorzugt eine gewisse Schließbewegung durchgeführt. Es ist zu erkennen, dass infolge der bei einem anfänglichen Überführen aus der Ruheposition (Figuren 1 und 2) in die Zwischenpositionen der Figuren 3 und 4 vorliegenden Winkelverhältnisse der Kniehebelmechanik eine relativ große Übersetzung vorliegt, so dass die getätigte Bedienelementverschwenkung eine relativ große Verlagerung der Koppelhülse 6 und damit der Instrumentenbranchen bei relativ geringer Kraftübertragung bewirkt hat.

Die Figuren 5 und 6 zeigen das System nach Erreichen der Arbeitsposition. Der Kniehebelwinkel a ist nahezu völlig gestreckt und beträgt nahezu 180°. Das Verschwenken des Bedienelements 32 aus den Zwischenstellungen der Figuren 3 und 4 in die Arbeitsstellung hat aufgrund der dabei vorliegenden Winkelverhältnisse der Kniehebelmechanik bei einem relativ großen Verstellweg des Bedienelements 32 nur noch eine relativ geringe Axialverschiebung der Koppelhülse 6 in Richtung proximal und damit geringe Lageänderung der Instrumentenbranchen geführt. Allerdings ist das Verhältnis der auf das Bedienelement 32 durch einen Anwender aufgebrachten Betätigungskraft zur auf die Koppelhülse 6 wirkenden Kraft (und damit Schließkraft der Instrumentenbranchen) relativ klein (d.h. es können mit relativ geringen Betätigungskräften hohe Schließkräfte erzeugt werden).

Es ist gut zu erkennen, dass kurz vor Erreichen der Arbeitsposition die in das Kniehebelelement 30 integrierte Überlastsicherung in Form des Federabschnitts 34 seine volle Wirkung entfaltet, während ein Einfedern der beiden Federarme 35 und 36 in der Ruhestellung (Fig. 1, 2) infolge der dann vorliegenden Winkelverhältnisse ungünstig ist. Die integrierte Überlastsicherung entfaltet ihre (volle) Wirkung daher erst kurz vor Erreichen der Arbeitsstellung und hat in einem Verstellbereich nahe der Ruhestellung nur geringe oder keine Wirkung (aufgrund von Reibung zwischen bzw. Verkanten von Schraubbolzen 37 und Federarm 36), so dass dort ein direktes Ansprechverhalten gegeben ist. Die vom Federelement 18 auf das Bedienelement 32 zwangsläufig wirkende Kraft (die ein Rückstellen des Instruments aus der Arbeitsstellung in die Ruhestellung unterstützen soll) wird in vorteilhafter Weise in einem Bereich nahe der Arbeitsstellung durch die dort vorliegenden Winkelverhältnisse nur in einem geringen Maß, nahezu gar nicht, auf das Bedienelement übertragen, in einem Verstellbereich näher der Ruhestellung jedoch schon. Dies ist besonders vorteilhaft, da der Anwender in der Arbeitsstellung (oder nahe der Arbeitsstellung) keine oder allenfalls eine nur geringe Kraft zum Überwinden der Rückstellkraft des Federelements 18 aufbringen muss, da dessen Rückstellkraft über das Kniehebelelement 30 als Übertragungselement 30 in die Betätigungsmechanik und den Koppelmechanismus eingeleitet wird.

### Bezugszeichenliste

- 1: Instrument
- 2: Gehäuse
- 3: Griffelement, Handgriff
- 4: Schaft
- 5: Zug-Element, Zug-Band
- 6: Koppelhülse
- 7: distales Ende von 6
- 8: proximales Ende von 6
- 9: Koppelnase
- 10: Koppelöffnung
- 11: distaler Führungsabschnitt
- 12: proximaler Endabschnitt
- 13: Führungsschräge
- 14: aufgeweiteter Mittelabschnitt
- 15: Hohlraum
- 16: proximales Ende von 5
- 17: Nut
- 18: Federelement
- 19: Führung
- 20: Führungsschlitz
- 21: erster Federarm
- 22: zweiter Federarm
- 23: gabelförmige Aufnahme
- 24: Gabelarm
- 25: Gabelarm
- 26: zentrale Ausnehmung
- 27: Lagerbock
- 28: Lagerbock
- 29: Zapfen
- 30: federelastisches Übertragungselement, Kniehebelelement/Übersetzungseinheit
- 31: Lagerkontur
- 32: Bedienelement
- 33: Hebelarm
- 34: Federabschnitt
- 35: erster Federarm
- 36: zweiter Federarm
- 37: Stellschraube
- 38: U-förmige Aufnahme
- 39: Koppelaussparung
- 40: Koppelaussparung
- 41: Lagerstruktur, Schwenklager
- 42: Koppelarm/Kniehebelarm
- 43: Koppelarm/Kniehebelarm
- 44: zentrale Ausnehmung
- 45: Aufnahme an Bedienelement für Kniehebelelement
- 46: erster Kniehebelarm
- 47: zweiter Kniehebelarm
- a: Kniehebelwinkel
- A: Axialrichtung, Längsachse

## Patentansprüche

1. Chirurgisches Instrument (1), insbesondere elektrochirurgisches Instrument, mit einem Instrumentenschaft (4),
zwei daran distal angeordneten zueinander in einer Arbeitsstellung und einer Ruhestellung relativpositionierbaren Instrumentenbranchen,
einem Griffelement (3), an dem ein Bedienelement (32) zum Positionieren der Instrumentenbranchen beweglich angeordnet ist,
und einem Koppelmechanismus (4, 5, 6, 18, 30, 32, 34) mit einer eine Bewegung des Bedienelements (32) nicht linear in eine Relativbewegung zumindest einer der beiden Instrumentenbranchen wandelnden Übersetzungseinheit (18, 30, 32, 34),
wobei der Koppelmechanismus (4, 5, 6, 18, 30, 32, 34) ein Vorspannelement (18, 34) einer ersten Art aufweist, das ihn in die Ruhestellung und/oder in die Arbeitsstellung der Instrumentenbranchen vorspannt, wobei das Bedienelement (32) und das zumindest eine Vorspannelement (34) einer ersten Art derart ausgebildet und zueinander konfiguriert sind, dass sie eine kniehebelartige Übersetzungseinheit (18, 30, 32, 34) ausbilden, in welchem das Vorspannelement (34) in den Kraftübertragungszug des Koppelmechanismus (4, 5, 6, 18, 30, 32, 34) seriell eingefügt ist, wobei das chirurgische Instrument (1) neben dem Vorspannelement (34) der ersten Art zumindest ein zweites Vorspannelement (18) einer zweiten Art aufweist, das parallel zum Kraftübertragungszug sowie auf der in Kraftflussrichtung des Kraftübertragungszugs dem Bedienelement (32) abgewandten Seite der Übersetzungseinheit (18, 30, 32, 34) angeordnet ist, und der Koppelmechanismus (4, 5, 6, 18, 30, 32, 34) mit dem Vorspannelement (18) der zweiten Art in die Ruhestellung vorgespannt ist.

2. Chirurgisches Instrument (1) nach Anspruch 1, wobei das Bedienelement (32) einen ersten Kniehebelarm (46) hat/ausbildet und das zumindest eine Vorspannelement (34) der ersten Art einen zweiten Kniehebelarm (47) hat/ausbildet, der mit dem ersten Kniehebelarm (46) schwenkgekoppelt ist, um eine Schwenkbewegung des Bedienelements (32) in eine Translationsbewegung mit nicht-linearem Übersetzungsverhältnis entsprechend dem aktuellen Winkel zwischen dem ersten Kniehebelarm (46) und dem zweiten Kniehebelarm (47) zu transformieren.

3. Chirurgisches Instrument (1) nach Anspruch 2, wobei das zumindest eine Vorspannelement (34) der ersten Art eine vorzugsweise U-oder V-förmig gebogene Blattfeder ist, die zwei Federarme (35, 36) ausbildet, von denen der eine Federarm (36) am ersten Kniehebelarm (46) des Bedienelements (32) ausscharniert ist und der andere Federarm (35) mit einem Koppelelement (6), welches über ein Zug-Element (5) mit zumindest einer der beiden Instrumentenbranchen verbunden ist, wirkverbunden ist.

4. Chirurgisches Instrument (1) nach Anspruch 3, wobei der eine Federarm (36) ein mittig angeordnetes Durchgangs-/ Führungsloch und der andere Federarm (35) ein mittig angeordnetes Schraubengewindeloch hat, wobei ein Schraubenbolzen (37) mit Kopf durch das Durchgangsloch hindurchgeführt und in das Schraubenloch eingedreht ist, um den Abstand der beiden Federarme (35, 36) in Konstruktionslage federelastisch einzustellen.

5. Chirurgisches Instrument (1) nach Anspruch 3, wobei für die federelastische Einstellung der beiden Federarme (35, 36) in Konstruktionslage ein zusätzliches Einlegeelement, vorzugsweise ein U-geformtes Blech, oder Versteifungen vorgesehen sind.

6. Chirurgisches Instrument (1) nach Anspruch 3, wobei das zumindest eine Vorspannelement (18) der zweiten Art eine Druckfeder ist, die an dem Griffelement (3) gelagert ist, mit dem Koppelelement (6) gekoppelt ist, welches über das Zug-Element (5) mit zumindest einer der beiden Instrumentenbranchen verbunden ist, und axial zum Koppelelement (6), an einem proximalen Abschnitt des Koppelelements (6), angelenkt ist.

7. Chirurgisches Instrument (1) nach Anspruch 3, wobei das zumindest eine Vorspannelement (18) der zweiten Art eine Blattfeder ist, die einerseits an dem Griffelement (3) gelagert ist, und andererseits mit dem Koppelelement (6) gekoppelt ist, welches über das Zug-Element (5) mit zumindest einer der beiden Instrumentenbranchen verbunden ist.

8. Chirurgisches Instrument (1) nach Anspruch 6 oder 7, wobei das zumindest eine Vorspannelement (18) der zweiten Art zur Kopplung mit dem Koppelelement (6) eine insbesondere gabelförmige Aufnahme (23) aufweist, die vorzugsweise aus zwei beiderseits einer zentralen Ausnehmung (26) angeordneten Gabelarmen (24, 25) ausgebildet ist.

9. Chirurgisches Instrument (1) nach Anspruch 7 oder 8, wobei das zumindest eine blattfederförmige Vorspannelement (18) der zweiten Art einen ersten Federarm (21), der mit Griffelement (2) verbunden ist, und einen zweiten Federarm (22), der mit dem Koppelelement (6) verbunden ist, aufweist, und ein zwischen dem ersten (21) und zweiten (22) Federarm liegender Biegebereich des zumindest einen Vorspannelements (18) der zweiten Art innenseitig an einem Lagerbock (28) des Griffelements (2) anliegt.

10. Chirurgisches Instrument (1) nach Anspruch 3, wobei das zumindest eine Vorspannelement (34) der ersten Art einerseits eine Lagerkontur (31) zur schwenkbaren Anlenkung an dem ersten Kniehebelarm (46) des Bedienelements (32) und andererseits einen Koppelabschnitt (38), insbesondere in Form einer im Wesentlichen U-förmigen Aufnahme, mit zwei beiderseits einer zentralen Ausnehmung ausgebildeten Koppelarmen (42, 43) aufweist zur Wirkverbindung mit dem Koppelelement (6) zu den Instrumentenbranchen.

11. Chirurgisches Instrument (1) nach Anspruch 3, wobei das zumindest eine Vorspannelement (34) der ersten Art einerseits eine Lagerkontur (31) zur schwenkbaren Anlenkung an dem ersten Kniehebelarm (46) des Bedienelements (32) und andererseits Stifte aufweist zur Wirkverbindung mit dem Koppelelement (6) zu den Instrumentenbranchen.

12. Chirurgisches Instrument (1) nach Anspruch 10, wobei das zumindest eine Vorspannelement (34) der ersten Art zur Kopplung mit dem zumindest einen Vorspannelement (18) der zweiten Art an den Koppelarmen (42, 43) jeweils eine U-förmige Koppelaussparung (39) aufweist, und/oder das Vorspannelement (18) der zweiten Art zur Kopplung mit dem Vorspannelement (34) der ersten Art an den Gabelarmen (24, 25) jeweils eine im Wesentlichen U-förmige Koppelaussparung (40) aufweist.

13. Chirurgisches Instrument (1) nach Anspruch 12, wobei die Koppelaussparungen (39) des Vorspannelements (34) der ersten Art jeweils auf der der zentralen Ausnehmung (44) zugewandten Innenseite der Koppelarme (42, 43) ausgebildet sind.

## Claims

1. A surgical instrument (1), particularly an electrosurgical instrument, comprising an instrument shaft (4),
two instrument branches that are arranged distally thereon and can be positioned relative to each other in a working position and a rest position,
a handle element (3) on which an operating element (32) is movably arranged for positioning the instrument branches,
and a coupling mechanism (4, 5, 6, 18, 30, 32, 34) having a translating unit (18, 30, 32, 34) that converts a movement of the operating element (32) non-linearly into a relative movement of at least one of the two instrument branches,
wherein the coupling mechanism (4, 5, 6, 18, 30, 32, 34) comprises one prestressing element (18, 34) of a first type that prestresses the mechanism into the rest position and/or into the working position of said instrument branches, wherein the operating element (32) and the at least one prestressing element (34) of a first type are formed and configured with respect to each other such that they form a knee-lever-type translating unit (18, 30, 32, 34) in which the prestressing element (34) is serially inserted in the flux of force of the coupling mechanism (4, 5, 6, 18, 30, 32, 34), wherein
the surgical instrument (1) includes, apart from the prestressing element (34) of the first type, at least a second prestressing element (18) of a second type that is preferably arranged in parallel to the flux of force as well as on the side of the translating unit (18, 30, 32, 34) facing away from the operating element (32) in the direction of the flux of force, and in that the coupling mechanism (4, 5, 6, 18, 30, 32, 34) is prestressed into the rest position by the prestressing element (18) of the second type.

2. The surgical instrument (1) according to claim 1, wherein the operating element (32) has/forms a first knee lever arm (46) and the at least one prestressing element (34) of the first type has/forms a second knee lever arm (47) which is pivotally coupled to the first knee lever arm (46) to transform a pivoting motion of the operating element (32) into a translating motion having a non-linear translation ratio corresponding to the current angle formed between the first knee lever arms (46) and the second knee lever arm (47).

3. The surgical instrument (1) according to claim 2, wherein the at least one prestressing element (34) of the first type is a leaf spring that is preferably bent in U or V shape and forms two spring arms (35, 36) the one spring arm (36) of which is hinged on the first knee lever arm (46) of the operating element (32) and the other spring arm (35) of which is operatively connected to a coupling element (6) that is connected to at least one of the two instrument branches via a tension element (5).

4. The surgical instrument (1) according to claim 3, wherein the one spring arm (36) includes a centrally arranged through-hole/guide hole and the other spring arm (35) includes a centrally arranged screw-thread hole, with a screw bolt (37) including a head being passed through the through-hole and being screwed into the screw hole to resiliently adjust the distance of the two spring arms (35, 36) in the design position.

5. The surgical instrument (1) according to claim 3, wherein an additional inserting element, preferably a U-shaped sheet metal, or reinforcements are provided in the design position for resilient adjustment of the two spring arms (35, 36).

6. The surgical instrument (1) according to claim 3, wherein the at least one prestressing element (18) of the second type is a compression spring that is supported on the handle element (3), is coupled to the coupling element (6) connected to at least one of the two instrument branches via the tension element (5) and is articulated, axially relative to the coupling element (6), to a proximal portion of the coupling element (6).

7. The surgical instrument (1) according to claim 3, wherein the at least one prestressing element (18) of the second type is a leaf spring which, on the one hand, is supported on the handle element (3) and, on the other hand, is coupled to the coupling element (6) that is connected to at least one of the two instrument branches via the tension element (5).

8. The surgical instrument (1) according to claim 6 or 7, wherein the at least one prestressing element (18) of the second type includes, for coupling to the coupling element (6), an especially forked seat (23) which is preferably formed of two fork arms (24, 25) arranged on both sides of a central recess (26).

9. The surgical instrument (1) according to claim 7 or 8, wherein the at least one leaf spring-shaped prestressing element (18) of the second type includes a first spring arm (21) connected to the handle element (2) and a second spring arm (22) connected to the coupling element (6), and in that a bending area of the at least one prestressing element (18) of the second type located between the first (21) and second (22) spring arms abuts on the inner face on a bearing bracket (28) of the handle element (2).

10. The surgical instrument (1) according to claim 3, wherein the at least one prestressing element (34) of the first type comprises, on the one hand, a bearing contour (31) for pivotable articulation to the first knee lever arm (46) of the operating element (32) and, on the other hand, a coupling portion (38), especially in the form of a substantially U-shaped seat, having two coupling arms (42, 43) formed on both sides of a central recess for operative connection with the coupling element (6) to the instrument branches.

11. The surgical instrument (1) according to claim 3, wherein the at least one prestressing element (34) of the first type comprises, on the one hand, a bearing contour (31) for pivotable articulation to the first knee lever arm (46) of the operating element (32) and, on the other hand, pins for operative connection with the coupling element (6) to the instrument branches.

12. The surgical instrument (1) according to claim 10, wherein the at least one prestressing element (34) of the first type comprises, for coupling to the at least one prestressing element (18) of the second type, a substantially U-shaped coupling recess (39) at each of the coupling arms (42, 43), and/or in that the prestressing element (18) of the second type comprises, for coupling to the prestressing element (34) of the first type, a substantially U-shaped coupling recess (40) at each of the fork arms (24, 25).

13. The surgical instrument (1) according to claim 12, wherein the coupling recesses (39) of the prestressing element (34) of the first type are formed on the inner face of each of the coupling arms (42, 43) facing the central recess (44).

## Revendications

1. Instrument chirurgical (1), en particulier instrument électrochirurgical, muni d'un manche de l'instrument (4),
de deux branches de l'instrument disposées distalement à celui-ci, positionnables de manière relative l'un par rapport à l'autre dans une position de travail et une position de repos,
un élément de saisie (3), sur lequel un élément de commande (32) est disposé de manière mobile pour le positionnement des branches de l'instrument,
et un mécanisme de couplage (4, 5, 6, 18, 30, 32, 34) muni d'une unité de transmission (18, 30, 32, 34) convertissant de manière non linéaire un mouvement de l'élément de commande (32) en un mouvement relatif d'au moins l'une des deux branches de l'instrument,
dans lequel le mécanisme de couplage (4, 5, 6, 18, 30, 32, 34) présente un dispositif de précontrainte (18, 34) d'un premier type, qui précharge celui-ci dans la position de repos et/ou la position de travail des branches de l'instrument, dans lequel l'élément de commande (32) et l'au moins un élément de précontrainte (34) d'un premier type sont disposés et configurés ensemble de telle sorte qu'ils forment une unité de transmission (18, 30, 32, 34) de type levier à genouillère, dans lequel l'élément de précontrainte (34) est inséré en série dans la chaîne de transmission du mécanisme de couplage (4, 5, 6, 18, 30, 32, 34), dans lequel
l'instrument chirurgical (1) présente, outre l'élément de précontrainte (34) du premier type, au moins un deuxième élément de précontrainte (18) d'un deuxième type, qui est disposé parallèlement à la chaîne de transmission, mais également sur le côté opposé à l'élément de commande (32) dans la direction du flux de force de la chaîne de transmission de l'unité de transmission (18, 30, 32, 34), et le mécanisme de couplage (4, 5, 6, 18, 30, 32, 34) est préchargé dans la position de repos avec l'élément de précontrainte (18) du deuxième type.

2. Instrument chirurgical (1) selon la revendication 1, dans lequel l'élément de commande (32) présente/forme un premier levier à genouillère (46) et l'au moins un élément de précontrainte (34) du premier type présente/forme un deuxième levier à genouillère (47), qui est couplé en rotation avec le premier levier à genouillère (46), afin de transformer un mouvement de rotation de l'élément de commande (32) en un mouvement de translation, selon un rapport de transmission non linéaire correspondant à l'angle actuel entre le premier levier à genouillère (46) et le deuxième levier à genouillère (47).

3. Instrument chirurgical (1) selon la revendication 2, dans lequel l'au moins un élément de précontrainte (34) du premier type est un ressort à lame pliée de préférence en U ou en V, qui forme deux bras de ressort (35, 36), dont l'un est un bras de ressort (36) articulé sur le premier levier à genouillère (46) de l'élément de commande (32) et l'autre bras de ressort (35) est connecté de manière fonctionnelle à un élément de couplage (6), lui-même connecté à au moins l'une des deux branches de l'instrument par l'intermédiaire d'un élément de traction (5).

4. Instrument chirurgical (1) selon la revendication 3, dans lequel le premier bras de ressort (36) a un trou traversant / de guidage disposé au centre et l'autre bras de ressort (35) a un trou fileté disposé au centre, dans lequel un boulon à vis (37) avec une tête passe à travers le trou traversant, et est vissée dans le trou de vis, afin d'ajuster la distance entre les deux bras de ressort (35, 36) en position de construction d'une manière élastique.

5. Instrument chirurgical (1) selon la revendication 3, dans lequel un élément d'insertion supplémentaire, de préférence une feuille de métal en forme de U, ou des renforts sont prévus pour le réglage élastique des deux bras de ressort (35, 36) en position de construction.

6. Instrument chirurgical (1) selon la revendication 3, dans lequel l'au moins un élément de précontrainte (18) du deuxième type est un ressort de compression qui est monté sur l'élément de saisie (3), qui est couplé à l'élément de couplage (6), lequel est relié par l'intermédiaire de l'élément de traction (5) à au moins l'une des deux branches de l'instrument et articulé axialement à l'élément de couplage (6) sur une portion proximale de l'élément de couplage (6).

7. Instrument chirurgical (1) selon la revendication 3, dans lequel l'au moins un élément de précontrainte (18) du deuxième type est un ressort à lame qui est monté d'une part sur l'élément de saisie (3), et d'autre part est couplé à l'élément de couplage (6), qui est relié par l'intermédiaire de l'élément de traction (5) à au moins l'une des deux branches de l'instrument.

8. Instrument chirurgical (1) selon la revendication 6 ou 7, dans lequel l'au moins un élément de précontrainte (18) du deuxième type présente, pour le couplage avec l'élément de couplage (6), un réceptacle en forme en particulier de fourche (23), qui est de préférence constitué de deux bras de fourche (24, 25) disposés des deux côtés d'une cavité centrale (26).

9. Instrument chirurgical (1) selon la revendication 7 ou 8, dans lequel l'au moins un élément de précontrainte en forme de ressort à lame (18) du deuxième type présente un premier bras de ressort (21) qui est relié à l'élément de saisie (2), et un deuxième bras de ressort (22) qui est relié à l'élément de couplage (6), et une zone de flexion de l'au moins un élément de précontrainte (18) du deuxième type, située entre les premier (21) et deuxième (22) bras de ressort, repose à l'intérieur sur un bloc de support (28) de l'élément de saisie (2).

10. Instrument chirurgical (1) selon la revendication 3, dans lequel l'au moins un élément de précontrainte (34) du premier type présente d'une part un contour de support (31) pour une articulation pivotante sur le premier levier à genouillère (46) de l'élément de commande (32), et d'autre part une section de couplage (38), en particulier sous forme d'un réceptacle sensiblement en forme de U, avec deux bras de couplage (42, 43) montés des deux côtés d'une cavité centrale, pour la liaison fonctionnelle avec l'élément de couplage (6) aux branches de l'instrument.

11. Instrument chirurgical (1) selon la revendication 3, dans lequel l'au moins un élément de précontrainte (34) du premier type présente d'une part un contour de support (31) pour une articulation pivotante sur le premier levier à genouillère (46) de l'élément de commande (32), et d'autre part des chevilles pour la liaison fonctionnelle avec l'élément de couplage (6) aux branches de l'instrument.

12. Instrument chirurgical (1) selon la revendication 10, dans lequel l'au moins un élément de précontrainte (34) du premier type pour le couplage avec l'au moins un élément de précontrainte (18) du deuxième type sur les bras de couplage (42, 43) présente chacun un renfoncement de couplage en forme de U (39) et/ou l'élément de précontrainte (18) du deuxième type pour le couplage avec l'élément de précontrainte (34) du premier type sur les bras de fourche (24, 25) présente chacun un renfoncement de couplage (40) sensiblement en forme de U.

13. Instrument chirurgical (1) selon la revendication 12, dans lequel les renfoncements de couplage (39) de l'élément de précontrainte (34) du premier type sont formés chacun du côté intérieur des bras de couplage (42, 43) tournés vers la cavité centrale (44).
